(19) Europäisches Patentamt
European Patent Office
Office européen des brevets

(11) **EP 2 332 969 B1**

(12) **EUROPÄISCHE PATENTSCHRIFT**

(45) Veröffentlichungstag und Bekanntmachung des Hinweises auf die Patenterteilung:
**07.08.2013 Patentblatt 2013/32**

(51) Int Cl.:
***C08G 83/00*** *(2006.01)*

(21) Anmeldenummer: **11158039.5**

(22) Anmeldetag: **15.11.2006**

(54) **Verwendung von hochfunktionellen, hoch- oder hyperverzweigten Polylysinen**

Use of highly functional, highly branched or hyperbranched polylysines

Application de polylysines hautement fonctionelles, très ou hyper-ramifiées

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **25.11.2005 DE 102005056592**

(43) Veröffentlichungstag der Anmeldung:
**15.06.2011 Patentblatt 2011/24**

(62) Dokumentnummer(n) der früheren Anmeldung(en) nach Art. 76 EPÜ:
**06819490.1 / 1 957 519**

(73) Patentinhaber: **BASF SE**
**67056 Ludwigshafen (DE)**

(72) Erfinder:
• **Bruchmann, Bernd**
**67251, Freinsheim (DE)**
• **Klok, Harm-Anton**
**1025, St. Sulpice (CH)**
• **Scholl, Markus Thomas**
**1007, Lausanne (CH)**

(56) Entgegenhaltungen:
| | |
|---|---|
| EP-A2- 0 331 616 | WO-A1-00/71601 |
| WO-A1-99/36781 | WO-A1-03/055935 |
| WO-A2-03/064452 | DE-A1- 2 322 310 |
| JP-A- 5 246 963 | JP-A- 11 255 892 |
| JP-A- 2003 128 589 | US-A- 5 137 874 |
| US-A- 5 932 539 | US-A1- 2002 025 919 |

• **VLASOV GP ET AL.: "Hyperbranched Poly(L-lysine) Containing Additional Amino Acids or Their Oligomers Between Branching Points: Synthesis and Structure", POLYMER SCIENCE, SER. A, Bd. 47, Nr. 5, Mai 2005 (2005-05), Seiten 422-429, XP008074529,**
• **RODRIGUEZ-HERNANDEZ JUAN ET AL: "Highly branched poly(L-lysine)", BIOMACROMOLECULES, ACS, WASHINGTON, DC, US, Bd. 4, Nr. 2, März 2003 (2003-03), Seiten 249-258, XP002250742, ISSN: 1525-7797**
• **JAGANNATH B. LAMTURE ET AL: "Intensely Luminescent Immunoreactive Conjugates of Proteins and Dipicolinate-Based Polymeric Tb(III) Chelates", BIOCONJUGATE CHEMISTRY, Bd. 6, Nr. 1, 1. Januar 1995 (1995-01-01), Seiten 88-92, XP55007184, ISSN: 1043-1802, DOI: 10.1021/bc00031a010**
• **LEA MA & LUKE A: "Effects of carbamoylation with alkyl isocyanates on the assay of proteins by dye binding", INTERNATIONAL JOURNAL OF PEPTIDE AND PROTEIN RESEARCH, Bd. 29, 1987, Seiten 561-567, XP000002659145,**
• **L GRAMMER ET AL: "Characterization of an antigen in acute anaphylactic dialysis reactions: Ethylene oxide-altered human serum albumin", JOURNAL OF ALLERGY AND CLINICAL IMMUNOLOGY, Bd. 76, Nr. 5, 1. November 1985 (1985-11-01), Seiten 670-675, XP55007174, ISSN: 0091-6749, DOI: 10.1016/0091-6749(85)90669-4**

EP 2 332 969 B1

- SAWHNEY A S ET AL: "Poly(ethylene oxide)-graft-poly(L-lysine) copolymers to enhance the biocompatibility of poly(L-lysine)-alginate microcapsule membranes", BIOMATERIALS, ELSEVIER SCIENCE PUBLISHERS BV., BARKING, GB, Bd. 13, Nr. 12, 1. Januar 1992 (1992-01-01), Seiten 863-870, XP024142619, ISSN: 0142-9612, DOI: 10.1016/0142-9612(92)90180-V [gefunden am 1992-01-01]

- MENZ TL & CHAPMAN T: "Synthesis and Characterization of Hyperbranched Polylysine", POLYMER PREPRINTS, Bd. 44, Nr. 2, 2003, Seiten 842-843, XP008074519, ISSN: 0032-3934

**Beschreibung**

Die vorliegende Erfindung betrifft bestimmte Verwendungen von bestimmten, modifizierten Polylysinen.

Sowohl in der Forschung als auch in der Industrie besteht ein zunehmendes Interesse an dendrimeren und hyperverzweigten Polypeptiden. Potentielle Anwendungen bestehen in biologisch-medizinischer Ausrichtung zum Beispiel in der Entwicklung neuer Multipler-Antigen-Peptide (MAPs), als Trägerplattform von Kontrastmitteln für die Kernresonanz-Bildgebung oder als Gentransporter.

Dendritische Polymere mit perfekt symmetrischer Struktur, sogenannte Dendrimere, lassen sich ausgehend von einem zentralen Molekül durch kontrollierte schrittweise Verknüpfung von jeweils zwei oder mehr di- oder mehrfunktionellen Monomeren mit jedem bereits gebundenen Monomer herstellen. Dabei wächst mit jedem Verknüpfungsschritt die Zahl der Monomerendgruppen (und damit der Verknüpfungen) an, und man erhält Polymere mit baumartigen Strukturen, im Idealfall kugelförmig, deren Äste jeweils exakt dieselbe Anzahl von Monomereinheiten enthalten. Aufgrund dieser perfekten Struktur sind die Polymereigenschaften vorteilhaft, beispielsweise beobachtet man eine überraschend geringe Viskosität und eine hohe Reaktivität aufgrund der hohen Anzahl funktioneller Gruppen an der Kugeloberfläche. Allerdings wird die Herstellung dadurch verkompliziert, dass bei jedem Verknüpfungsschritt Schutzgruppen eingeführt und wieder entfernt werden müssen und Reinigungsoperationen erforderlich sind, weshalb man Dendrimere üblicherweise nur im Labormaßstab herstellt.

Dendritische Polymere mit weniger perfekter Struktur, sogenannte hyperverzweigte Polymere oder "hyperbranched polymers", kann man dagegen mit großtechnischen Verfahren herstellen. Hyperverzweigte Polymere weisen neben perfekten dendrimeren Strukturen auch lineare Polymerketten und ungleiche Polymeräste auf, was jedoch die Polymereigenschaften verglichen zu denen der perfekten Dendrimere nicht wesentlich verschlechtert.

Hyperverzweigte Polymere lassen sich über die sogenannte AB2-Route herstellen. Als AB2-Molekül wird ein trifunktionelles Monomer bezeichnet, das eine reaktiven Gruppe A und zwei reaktive Gruppen B aufweist. Sind diese Gruppen A und B miteinander reaktiv, kann man durch intermolekulare Reaktion hyperverzweigte Polymere erzeugen.

Zur Definition von dendrimeren und hyperverzweigten Polymeren siehe auch P.J. Flory, J. Am. Chem. Soc. 1952, 74, 2718 und H. Frey et al., Chemistry - A European Journal, 2000, 6, No. 14, 2499.

Unter "hyperverzweigt" wird im Zusammenhang mit der vorliegenden Erfindung verstanden, dass der Verzweigungsgrad (Degree of Branching, DB) 10 bis 99,9 %, bevorzugt 20 bis 99 %, besonders bevorzugt 20 - 95 % beträgt.

Unter "dendrimer" wird im Zusammenhang mit der vorliegenden Erfindung verstanden, daß der Verzweigungsgrad 99,9 - 100% beträgt.

Der Verzweigungsgrad ist definiert als

$$DB\ [\%] = 100 * (T + Z) / (T + Z + L)$$

wobei T die mittlere Anzahl der terminalen Monomereinheiten, Z die mittlere Anzahl der verzweigten Monomereinheiten und L die mittlere Anzahl der linearen Monomereinheiten, bedeutet. Zur Definition des "Degree of Branching" siehe auch H. Frey et al., Acta Polym. 1997, 48, 30.

Unter hyperverzweigten Polypeptiden werden im Rahmen dieser Erfindung unvernetzte Makromoleküle aufgebaut aus Aminosäuren verstanden, die sowohl strukturell als auch molekular uneinheitlich sind. Sie können auf der einen Seite ausgehend von einem Zentral molekül analog zu Dendrimeren, jedoch mit uneinheitlicher Kettenlänge der Äste aufgebaut sein. Sie können auf der anderen Seite auch linear, mit funktionellen Seitengruppen, aufgebaut sein oder aber, als Kombination der beiden Extreme, lineare und verzweigte Molekülteile aufweisen.

Für die Synthese von hyperverzweigten Polylysinen sind drei prinzipielle Verfahren bekannt:

Verfahren 1 basiert auf der ringöffnenden Polymerisation von ε-geschützten L-Lysin-N-Carboxyanhydriden (NCAs) mit einem nukleophilen Starter,
Verfahren 2 verwendet an der Carboxylgruppe aktivierte Derivate von L-Lysin*2HCl,
Verfahren 3 beinhaltet die direkte thermische Polymerisation von L-Lysinen.

Verfahren 1, hyperverzweigte L-Lysin-Polymere basierend auf der ringöffnenden Polymerisation von ε-geschützten L-Lysin-N-Carboxyanhydriden:

Hyperverzweigte Poly(L-Lysine) wurden von Klok et al. (WO 2003/064452 und Macromolecules 2002, 35, 8718-8723) beschrieben. Orthogonal Nε-geschützte Butoxycarbonyl-L-Lysin (Boc-Lysin; = temporäre Schutzgruppe) und ε-Benzyloxycarbonyl-L-Lysin (Z-Lysin; = permanente Schutzgruppe) NCAs wurden mit einem aliphatischen Amin (z.B. Hexylamin) als Starter ringöffnend polymerisiert. Die temporäre Schutzgruppe wurde mit Trifluoressigsäure (TFA) entfernt, und die freien Aminogruppen konnten als weiterer Starter für eine neue Polymerisation dienen. Im letzten Schritt wurden die Z-Schutzgruppen mit Bromwasserstoff/Essigsäure (HBr/AcOH) abgespalten.

Weiterhin wurden hyperverzweigte Poly(L-Lysine) von Rodriguez-Hernández et al. (Biomacromolecules2003, 4, 249-258) beschrieben. Eine Mischung aus $N^\varepsilon$-Trifluoroacetyl-L-Lysin-NCA (TFA-Lys-NCA) und Z-Lysin-NCA wurden mit einem aliphatischen Amin ringöffnend polymerisiert. In einem separaten Kupplungsschritt wurde $N^\alpha$,$N^\varepsilon$-di(9-fluorenylmethoxycarbonyl)-L-Lysin ($N^\alpha$,$N^\varepsilon$-diFmoc Lys) als Verzweigungspunkt eingebracht. Entschützen mit Piperidin in DMF ergab zwei neue Amingruppen die eine ringöffnende Polymerisation von TFA-Lys-NCA und Z-Lys-NCA ermöglichen. Diese Reaktionszyklen wurden mehrfach wiederholt. Strukturell ähnliche, hyperverzweigte Blockcopolymere wurden auch von Birchall et al. beschrieben (Chem. Commun. 1998, 1335-1336). $\alpha$-Aminosäure-NCAs wurden mit einem aliphatischen Amin ringöffnend polymerisiert. $N,N'$-di(benzyloxycarbonyl)L-Lysin p-nitrophenylester wurde als Verzweigungspunkt eingebracht, der nach Entschützen mit $H_2$/Pd/C zwei freie Amingruppen zur weiteren Ringöffnung von Aminosäure-NCAs hatte. Diese Reaktionszyklen wurden mehrfach wiederholt.

Nachteilig an all diesen Reaktionsführungen ist, daß Schutzgruppen erforderlich sind, was die Reaktion wesentlich erschwert.

Verfahren 2, hyperverzweigte L-Lysin-Polymere basierend auf an der Carboxylgruppe aktivierte Derivate von L-Lysin*2HCl.

Hyperverzweigte Polylysine wurden in einer Eintopfsynthese unter Aktivierung der Carboxylgruppe mittels N-Hydroxysuccinimid (NHS) hergestellt. NHS-aktiviertes L-Lysin*2HCl wurde in Dimethylsulfoxyd (DMSO) unter Zugabe von katalytischen Mengen an Dimethylaminopyridin (DMAP) und 3 Äquivalenten an Diisopropylethylamin (DIEA) 23 Stunden gerührt und das Polymer in Ethylacetat ausgefällt. Das Polymer hatte ein Molekulargewicht von Mw = 5100. Mit den gleichen Reagenzien wurden in einer "pseudo-schrittweisen" Polymerisation unter wiederholter Monomerzugabe Molekulargewichte von Mw = 8640 erreicht. Des Weiteren wurde das Monomer auch auf Tris(2-aminoethyl)amine als Kernmolekül aufpolymerisiert. Siehe dazu auch T.L. Menz und T. Chapman, Polym. Prep. 2003, 44(2), 842 -743.

Nachteilig an der von Menz offenbarten Reaktionsführung ist, daß die Carboxylfunktion durch ein Spezialreagenz aktiviert werden muß, was die Reaktionsführung kompliziert. Verfahren 3, thermische Copolymerisationen von Aminosäuremischungen: die thermische Polymerisation von freiem Lysin ist bekannt und wurde unter verschiedenen Reaktionsbedingungen durchgeführt.

Plaquet und Mitarbeiter (Biochimie 1975, 57 1395-1396) polymerisierten L-Lysin in wässriger Lösung bei 105 °C über einen Zeitraum von bis zu 10 Wochen, oder aber durch Erhitzen auf 165 °C für 8 Stunden. Die Reaktion wurde ohne Katalysator durchgeführt und die Ausbeuten waren mit durchweg unter 72,5 % sehr niedrig.

Harada (Bull. Chem. Soc. Japan 1959, 32, 1007-1008) polymerisierte L-Lysin bei 180 bis 230 °C zwischen 30 Minuten und 2 Stunden unter Stickstoffatmosphäre. Bei einer Umsetzung unterhalb von 180 °C wird lediglich die Bildung von Lactamen berichtet. Über Molekulargewicht oder Struktur wird nichts berichtet. Die erhaltenen Homopolymere weisen einen deutlichen Gelanteil auf. Die Homopolymerisation von Lysin-Hydrochlorid wurde nicht erreicht (S. 1008, linke Spalte unten).

Rohlfing und Mitarbeiter (Archives of Biochemistry and Biophysics 1969, 130, 441-448) polymerisierten L-Lysin (freie Base) unter Stickstoffatmosphäre zwischen 186 und 192 °C. Sie erreichten Molekulargewichte von bis zu 3600 Da und höher. Es wurden hier auch verzweigte Anteile vermutet (siehe dazu Vergleichsversuch 11). Die von Rohlfing et al. beschriebenen Molekulargewichte > 100.000 konnten im Vergleichsversuch nicht gefunden werden.

WO 00/71600 beschreibt die Kondensation von L-Lysin-Monohydrat in einer Druckapparatur. Die Molekulargewichte der erhaltenen Homopolymere sind gering. Die Kondensation der freien Lysin-Base führt zu vernetzten Kondensationsprodukten und wird entweder unkatalysiert oder durch Mineralsäuren oder deren Salze katalysiert durchgeführt. Hydrochloride müssen vor der Umsetzung mit einem Äquivalent Base in die freie Base überführt werden, bevor sie gemäß der WO 00/71600 umgesetzt werden können.

Fox et al. (BioSystems 1976, 8, 40-44) verwendeten sowohl L-Lysin, als auch L-Lysin*HCl als Ausgangsmonomer für die thermische Polymerisation bei 195°C. Hierbei wurde bei Verwendung von L-Lysin bei 170°C Reaktionstemperatur das cyclische Lactam erhalten. L-Lysin*HCl konnte lediglich unter Zusatz von Orthophosphorsäure bei 195°C zur Reaktion gebracht werden. Die dabei erhaltenen Molekulargewichte waren gering (siehe Vergleichsversuch 12).

In der JP 11-255892 A werden acylierte Polylysine beschrieben, welche sowohl in Wasser als auch in organischen Lösungsmitteln löslich sind und die als biologisch abbaubare Tenside eingesetzt werden können. Das Molekulargewicht kann dabei 1000 bis 5000 betragen.

Aufgabe der vorliegenden Erfindung war es, Verwendungen von durch ein einfaches Verfahren herstellbaren, modifizierten Polylysinen zur Verfügung zu stellen, in dem auf Schutzgruppenoperationen und Aktivierung von Carboxylgruppen verzichtet werden kann und in dem auch höhere Molekulargewichte als aus dem Stand der Technik bekannt erreicht werden können.

Die Aufgabe wurde gelöst durch die Verwendung von modifizierten Polylysinen, erhältlich durch Umsetzung eines beliebig erhaltenen Polylysins mit einem gewichtsmittleren Molekulargewicht $M_w$ von mehr als 7.500 Da und bis zu 750.000 Da mit mindestens einer Säuregruppen enthaltenden Verbindung, wobei diese Säuregruppen enthaltende Verbindung mindestens eine saure Gruppe, ausgewählt aus der Gruppe bestehend aus Carboxyl-, Sulfonsäure-, Sulfat- und Phosphonsäuregruppen, aufweist und mindestens einen Substituenten trägt, ausgewählt aus der Gruppe bestehend aus linearen oder verzweigten Alkylresten, gegebenenfalls substituierten Cycloalkylresten und gegebenenfalls substituierten Arylresten, die jeweils bis zu 20 Kohlenstoffatome aufweisen können, als Haftvermittler, Thixotropiermittel, Löslichkeitsvermittler (Solubilisatoren), als Phasentransferreagenz für in Wasser unlösliche Chemikalien, als Phasentransferreagenz für in Wasser lösliche Chemikalien oder als Surface-Modifier.

Die Polylysine können hergestellt werden durch ein Verfahren zur Herstellung von unvernetzten hyperverzweigten Polylysinen, in dem man

- (A) ein Salz von Lysin mit mindestens einer Säure,
- (B) gegebenenfalls mindestens eine andere Aminosäure als Lysin,
- (C) gegebenenfalls mindestens eine Di- oder Polycarbonsäure oder deren copolymerisationsfähige Derivate und
- (D) gegebenenfalls mindestens ein Di- oder Polyamin oder deren copolymerisationsfähige Derivate,
- (E) gegebenenfalls in mindestens einem Lösungsmittel
  bei einer Temperatur von 120 bis 200 °C umsetzt
- in Gegenwart mindestens eines Katalysators (F) ausgewählt aus der Gruppe bestehend aus

  -- (F1) tertiäre Amine und Amidine,
  -- (F2) basische Alkalimetall-, Erdalkalimetall- oder quartäre Ammoniumsalze und
  -- (F3) Alkanolate, Alkanoate, Chelate oder metallorganische Verbindungen der Metalle der Gruppen IIIA bis VIIIA oder IB bis VB im Periodensystem der Elemente.

Mit dem Verfahren ist es möglich unvernetzte hyperverzweigte Polylysine mit einem gewichtsmittleren Molekulargewicht $M_w$ bis zu 750.000 Da, bevorzugt bis zu 700.000 Da, besonders bevorzugt bis zu 650.000 Da, ganz besonders bevorzugt bis zu 600.000 Da und insbesondere bis zu 550.000 Da herzustellen.

Durch die Reaktionsführung ist es erstmals auch möglich, unvernetzte hyperverzweigte Polylysine mit einem gewichtsmittleren Molekulargewicht $M_w$ von mehr als 7.500 Da, besonders bevorzugt mehr als 10.000 Da, ganz besonders bevorzugt mehr als 12.000 Da, insbesondere mehr als 15.000 Da, speziell mehr als 20.000 Da und sogar mehr als 25.000 Da herzustellen.

Derartige, ausschließlich aus Komponente (A) mit gegebenenfalls Komponente (B) aufgebaute Polylysine zeichnen sich durch eine Wasserlöslichkeit bei 50 °C von mehr als 90 Gew% bei einem Molgewicht von 5.000 Da aus, bevorzugt mehr als 6.000 Da und besonders bevorzugt mehr als 7.000 Da.

Die Glasübergangstemperatur Tg, bestimmt gemäß ASTM-Vorschrift D3418-03 über Differential Scanning Calorimetry, beträgt in der Regel von -20 bis 100 °C, bevorzugt von -10 bis 80 °C und besonders bevorzugt von 0 bis 60 °C.

Unter dem Begriff "unvernetzt" wird erfindungsgemäß verstanden, daß die aus einem Salz (A) von Lysin mit mindestens einer Säure erhaltenen Polylysine einen geringeren Vernetzungsgrad aufweisen als Polylysine, gleichen gewichtsmittleren Molekulargewichts $M_w$, die durch Polymerisation von freier Lysin-Base erhalten worden sind.

Ein Maß dafür ist beispielsweise ein Vergleich des Gelgehalts der Polylysine, d.h. der bei 24stündiger Lagerung bei Raumtemperatur (23 °C) unter Wasser unlösliche Anteil des Polylysins, dividiert durch die Gesamtmenge der Probe und multipliziert mit 100.

Bei den Polylysinen beträgt der Gelgehalt in der Regel nicht mehr als 20% verglichen mit solchen Polylysinen, die durch Polymerisation von freier Lysin-Base erhalten woden sind, bevorzugt nicht mehr als 10% und besonders bevorzugt nicht mehr als 5%.

Des weiteren ist die Verwendung von modifizierten Polylysinen ein Gegenstand der vorliegenden Erfindung.

Die Reaktion wird in der Regel bei einer Temperatur von 120 bis 200 °C durchgeführt, bevorzugt 130 bis 180 °C und besonders bevorzugt 150 bis 170 °C und ganz besonders bevorzugt 150 bis 160 °C.

Der Druck, bei dem die Reaktion durchgeführt wird, spielt eine untergeordnete Rolle. Wenn ein Lösungsmittel (E) eingesetzt wird, das einen niedrigeren Siedepunkt aufweist als die gewünschte Reaktionstemperatur, so ist es sinnvoll Druck anzulegen, damit die gewünschte Reaktionstemperatur erreicht werden kann.

Die Reaktionszeit variiert je nach gewünschtem Molekulargewicht und beträgt in der Regel mindestens eine Stunde, bevorzugt mindestens 2 Stunden, besonders bevorzugt mindestens 4 Stunden, ganz besonders bevorzugt mindestens 6 Stunden und insbesondere mindestens 8 Stunden. In der Regel ist die Reaktion nach nicht mehr als 72 Stunden abgeschlossen, bevorzugt nach nicht mehr als 60 Stunden, besonders bevorzugt nach nicht mehr als 48 Stunden und ganz besonders bevorzugt nach nicht mehr als 36 Stunden.

Je höher das gewünschte Molekulargewicht der Polylysine ist, desto länger muß man in der Regel die Reaktionszeit wählen.

Die Reaktion kann kontinuierlich oder bevorzugt diskontinuierlich durchgeführt werden. Dabei kann das Lysin-Edukt sowohl vollständig vorgelegt werden, als auch langsam kontinuierlich in den Reaktor gegeben werden. Letztere Fahrweise wird auch als "slow monomer addition" bezeichnet. Bevorzugt wird die Reaktion in einer sogenannten "Eintopffahrweise" durchgeführt, in der das Monomer vollständig vorgelegt und die Reaktion in einem rückvermischten Reaktor durchgeführt wird. Denkbar sind aber auch Reaktionsführungen in einem mehrstufigen Reaktorsystem, beispielsweise einer Rührkesselkaskade, oder einem Rohrreaktor. In einer bevorzugten alternativen Ausführungsform der vorliegenden Erfindung kann die Reaktion in einem Kneter, Extruder, Intensivmischer oder Schaufeltrockner durchgeführt werden.

Die Reaktion kann gegebenenfalls auch unter Zuhilfenahme von Ultraschall oder Mikrowellenstrahlung durchgeführt werden.

Die einzelnen Komponenten können zu Beginn der Reaktionsführung vorgelegt oder gestaffelt zugegeben werden, je nachdem, zu welchem Stadium der Polymerbildung man die jeweiligen Reaktionskomponenten in das Polymer einbauen will.

Lysin kann als Salz (A) der freien Lysinbase mit einer Säure eingesetzt werden, bevorzugt einer Säure mit einem pKs-Wert von weniger als 2,2, besonders bevorzugt einer starken Säure.

Beispiele für Säuren sind Essigsäure, Ameisensäure, Kohlensäure, Glykolsäure, Propionsäure oder Milchsäure.

Beispiel für Säuren mit einem $pK_s$-Wert von weniger als 2,2 sind beispielsweise Phosphorsäure ($H_3PO_4$), Phosphorige Säure ($H_3PO_3$), Pyrophosphorsäure ($H_4P_2O_7$) oder Hydrogensulfat ($HSO_4^-$).

Beispiele für starke Säuren sind Schwefelsäure ($H_2SO_4$), Perchlorsäure, Salzsäure, Bromwasserstoffsäure.

Ganz besonders bevorzugt sind Schwefelsäure und Salzsäure, insbesondere Salzsäure.

Die Bildung eines inneren Salzes bei Lysin zählt dabei nicht als Salzbildung, die Säure muß eine andere als Lysin sein.

Weiterhin ist es möglich, das Salz des Lysin als beliebiges Hydrat einzusetzen. Dabei ist es erfindungsgemäß nicht relevant, welches Hydrat eingesetzt wird.

Da Lysin zwei Aminogruppen aufweist, können bezogen auf die Menge an Lysin bevorzugt mehr als 50 mol%, besonders bevorzugt 50 bis 200 mol%, ganz besonders bevorzugt 75 bis 200 mol% und insbesondere 100 bis 200 mol% Säure zur Salzbildung eingesetzt werden.

Gegebenenfalls, wenn auch weniger bevorzugt, kann die Carboxygruppe des Lysin auch als Ester, beispielsweise als $C_1$-$C_{10}$-Alkylester, bevorzugt als $C_1$ - $C_4$- Alkylester vorliegen.

Lysin kann enantiomerenrein oder als Racemat eingesetzt werden, bevorzugt als Racemat oder in Form von L-Lysin, besonders bevorzugt in Form von L-Lysin.

Selbstverständlich kann L-Lysin auch mit anderen Aminosäuren (B) copolymerisiert werden. Genannt seien hier zum Beispiel Glycin, Alanin, β-Alanin, Valin, Leucin, Isoleucin, tert.-Leucin, Phenylalanin, Tyrosin, Tryptophan, Prolin, Asparaginsäure, Glutaminsäure, Asparagin, Glutamin, Serin, Threonin, Cystein, Methionin, Arginin, Histidin, 4-Aminobuttersäure, Cystin, Citrullin, Theanin, Homocystein, 4-Hydroxyprolin, Alliin oder Ornithin.

Aminosäuren (B) sind dabei Aminosäuren, die mindestens eine primäre oder sekundäre Aminogruppe und mindestens eine Carboxylgruppe aufweisen.

Weiterhin können die unvernetzten hyperverzweigten Polylysine auch mit Carbonsäuren (C) oder Aminen (D) statistisch oder block-copolymerisiert werden, wobei darauf zu achten ist, dass das gesamte molare Verhältnis von Aminogruppen zu Carboxylgruppen im Reaktionsgemisch zwischen 3:1 bis 1:3 liegt, bevorzugt 3:1 bis 1:2, besonders bevorzugt 3:1 bis 1:1 und ganz besonders bevorzugt 2,5:1 bis 1,5:1.

Für diese Copolymerisation geeignete Di- und Polycarbonsäuren (C) weisen üblicherweise mindestens 2, bevorzugt 2 bis 4, besonders bevorzugt 2 bis 3 und ganz besonders bevorzugt 2 Carboxylgruppen auf. Bevorzugte Di- und Polycarbonsäuren (C) enthalten 2 bis 30 Kohlenstoffatome und können aliphatisch, cycloaliphatisch oder aromatisch sein.

Als Dicarbonsäuren kommen z.B. in Betracht: Oxalsäure, Malonsäure, Bernsteinsäure, Glutarsäure, Adipinsäure, Pimelinsäure, Korksäure, Azelainsäure, Sebacinsäure, Undecan-α,ω-dicarbonsäure, Dodecan-α,w-dicarbonsäure, cis- und trans-Cyclohexan-1,2-dicarbonsäure, cis- und trans-Cyclohexan-1,3-dicarbonsäure, cis- und trans-Cyclohexan-1,4-dicarbonsäure, cis- und trans-Cyclopentan-1,2-dicarbonsäure sowie cis- und trans-Cyclopentan-1,3-dicarbonsäure, wobei die Dicarbonsäuren substituiert sein können mit einem oder mehreren Resten, ausgewählt aus:

$C_1$-$C_{10}$-Alkylgruppen, beispielsweise Methyl, Ethyl, n-Propyl, iso-Propyl, n-Butyl, isoButyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, 2-Ethylhexyl, n-Nonyl oder n-Decyl,

$C_3$-$C_{12}$-Cycloalkylgruppen, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl,

Alkylengruppen wie Methylen oder Ethyliden, und/oder

C$_6$-C$_{14}$-Arylgruppen wie beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl.

Als Beispiele für substituierte Dicarbonsäuren seien genannt: 2-Methylmalonsäure, 2-Ethylmalonsäure, 2-Phenylmalonsäure, 2-Methylbernsteinsäure, 2-Ethylbernsteinsäure, 2-Phenylbernsteinsäure, Itaconsäure und 3,3-Dimethylglutarsäure.

Ebenso sind aromatische Dicarbonsäuren wie beispielsweise Phthalsäure, Isophthalsäure oder Terephthalsäure, geeignet.

Als Tricarbonsäuren bzw. Tetracarbonsäuren eignen sich z.B. Trimesinsäure, Trimellitsäure, Pyromellitsäure, Butantricarbonsäure, Naphthalentricarbonsäure und Cyclohexan-1,3,5-tricarbonsäure.

Ausgeschlossen als Komponente (C) sind solche Di- oder Polycarbonsäuren, die aktivierte Doppelbindungen, wie beispielsweise $\alpha,\beta$-ethylenisch ungesättigte Doppelbindungen aufweisen und/oder Aminosäuren (B) sind.

Bevorzugt als Komponente (C) sind solche Di- oder Polycarbonsäuren, die außer Carboxylgruppen keine weiteren funktionellen Gruppen aufweisen.

Weiterhin lassen sich Gemische von zwei oder mehreren der vorgenannten Carbonsäuren einsetzen. Die Carbonsäuren können entweder als solche oder in Form von Derivaten verwendet werden. Solche Derivate sind insbesondere

- die Anhydride der genannten Carbonsäuren, und zwar in monomerer oder auch polymerer Form;
- die Ester der genannten Carbonsäuren, z.B.

  • Mono- oder Dialkylester, bevorzugt C$_1$ bis C$_4$-Alkylester, besonders bevorzugt Mono- oder Dimethylester oder die entsprechenden Mono- oder Diethylester, aber auch die von höheren Alkoholen wie beispielsweise n-Propanol, iso-Propanol, n-Butanol, Isobutanol, tert.-Butanol, n-Pentanol, n-Hexanol abgeleiteten Mono- und Dialkylester,
  • Mono- und Divinylester sowie
  • gemischte Ester, bevorzugt Methyl ethylester.

Man kann auch ein Gemisch aus einer Carbonsäure und einem oder mehreren ihrer Derivate, oder ein Gemisch mehrerer verschiedener Derivate von einer oder mehreren Dicarbonsäuren, einsetzen.

Besonders bevorzugt setzt man als Carbonsäure Bernsteinsäure, Glutarsäure, Adipinsäure, Phthalsäure, Isophthalsäure, Terephthalsäure oder deren Mono- oder Dimethylester ein.

Geeignete Amine (D) weisen üblicherweise mindestens 2, bevorzugt 2 bis 6, besonders bevorzugt 2 bis 4 Aminogruppen, weisen in der Regel 2 bis 30 C-Atomen auf und können aliphatisch, cycloaliphatisch oder aromatisch sein. Die Amine (D) weisen primäre und/oder sekundäre Aminogruppen auf.

Als Diamine kommen bevorzugt solche der Formel R$^1$-NH-R$^2$-NH-R$^3$ in Betracht, worin R$^1$ und R$^3$ unabhängig voneinander Wasserstoff oder einen Alkylrest, Cycloalkylrest, Arylrest oder Arylalkylrest mit 1 bis 20 C-Atomen bedeuten. Der Alkylenest R$^2$ kann linear oder auch cyclisch sein.

Bevorzugte Diamine sind beispielsweise Ethylendiamin, die Propylendiamine (1,2-Diaminopropan and 1,3-Diaminopropan), N-Methyl-ethylendiamin, Piperazin, Tetramethylendiamin (1,4-Diaminobutan), N,N'-Dimethylethylendiamin, N-Ethylethylendiamin, 1,5-Diaminopentan, 1,3-Diamino-2,2-diethylpropan, 1,3-Bis(methylamino)propan, Hexamethylendiamin (1,6-Diaminohexan), Heptandiamin, Octandiamin, Nonandiamin, Decandiamin, Dodecandiamin, Hexadecandiamin, Toluylendiamin, Xylylendiamin, Diaminodiphenylmethan, Diaminodicyclohexylmethan, Phenylendiamin, Cyclohexylendiamin, Bis(aminomethyl)cyclohexan, Diaminodiphenylsulfon, 1,5-Diamino-2-methylpentan, 3-(Propylamino)-propylamin, N,N'-Bis-(3-aminopropyl)-piperazin, N,N'-Bis-(3-aminopropyl)-piperazin, Isophorondiamin (IPDA), 3 (bzw. 4), 8 (bzw. 9)-Bis(aminomethyl)-tricyclo[5.2.1.0$^{2.6}$]decan-Isomerengemische, 2-Butyl-2-ethyl-1,5-pentamethylendiamin, 2,2,4- oder 2,4,4-Trimethyl-1,6-hexamethylendiamin, 2-Aminopropylcyclohexylamin, 3(4)-Aminomethyl-1-methylcyclohexylamin, 1,4-Diamino-4-methylpentan, Amin-terminierte Polyoxyalkylenpolyole (sogenannte Jeffamine) oder Amin-terminierte Polytetramethylenglykole.

Bevorzugt sind Butylendiamin, Pentandiamin, Hexamethylendiamin, Toluylendiamin, Xylylendiamin, Diaminodiphenylmethan, Diaminodicyclohexylmethan, Phenylendiamin, Cyclohexylendiamin, Diaminodiphenylsulfon, Isophorondiamin, Bis(aminomethyl)cyclohexan, Amin-terminierte Polyoxyalkylenpolyole (sogenannte Jeffamine) oder Amin-terminierte Polytetramethylenglykole

Geeignete Amine mit drei oder mehr reaktiven primären und/oder sekundären Aminogruppen sind beispielsweise Tris(aminoethyl)amin, Tris(aminopropyl)amin, Tris(aminohexyl)amin, Trisaminohexan, 4-Aminomethyl-1,8-octandiamin, Trisaminononan, Bis(aminoethyl)amin, Bis(aminopropyl)amin, Bis(aminobutyl)amin, Bis(aminopentyl)amin, Bis(aminohexyl)amin, N-(2-Aminoethyl)propandiamin, Melamin, oligomere Diaminodiphenylmethane (Polymer-MDA), N,N'-Bis(3-aminopropyl)ethylendiamin, N,N'-Bis(3-aminopropyl)butandiamin, N,N,N',N'-Tetra(3-aminopropyl)ethylendiamin, N,N,N',N'-Tetra(3-aminopropyl)-butylendiamin, drei- oder höherfunktionelle Amin-terminierte Polyoxyalkylenpolyole (sogenannte Jeffamine), drei- oder höherfunktionelle Polyethylenimine oder drei- oder höherfunktionelle Polypropylenimine.

Bevorzugte Amine mit drei oder mehr reaktiven primären und/oder sekundären Aminogruppen sind Tris(aminoethyl)amin, Tris(aminopropyl)amin, Tris(aminohexyl)amin, Trisaminohexan, 4-Aminomethyl-1,8-octandiamin, Trisaminononan, Bis(aminoethyl)amin, Bis(aminopropyl)amin, Bis(aminobutyl)amin, Bis(aminopentyl)amin, Bis(aminohexyl)amin, N-(2-Aminoethyl)propandiamin, Melamin oder drei- oder höherfunktionelle Amin-terminierte Polyoxyalkylenpolyole (sogenannte Jeffamine).

Besonders bevorzugt sind Amine mit drei oder mehr primären Aminogruppen, wie Tris(aminoethyl)amin, Tris(aminopropyl)amin, Tris(aminohexyl)amin, Trisaminohexan, 4-Aminomethyl-1,8-octandiamin, Trisaminononan oder drei- oder höherfunktionelle Amin-terminierte Polyoxyalkylenpolyole (sogenannte Jeffamine).

Man kann auch Gemische mehrerer Carbonsäuren bzw. Carbonsäurederivate, oder Gemische mehrerer Amine, verwenden. Dabei kann die Funktionalität der verschiedenen Carbonsäuren oder Amine gleich oder verschieden sein.

Die Umsetzung des Lysin (A) und ggf. der zusätzlichen Monomere (B) bis (D) kann optional in einem Lösungsmittel (E) erfolgen. Dabei können allgemein alle Lösungsmittel eingesetzt werden, bevorzugt solche, die gegenüber den jeweiligen Edukten unter den Reaktionsbedingungen inert sind. Bevorzugt wird in organischen Lösungsmitteln, wie Decan, Dodecan, Benzol, Toluol, Chlorbenzol, Dichlorbenzol, Xylol, Dimethylformamid, Dimethylacetamid oder Solventnaphtha gearbeitet. Denkbar und weiterhin bevorzugt sind aber auch Wasser und 1 bis 10 Kohlenstoffatome aufweisende Alkanole, insbesondere Methanol, Ethanol, *iso*-Propanol, n-Butanol und 2-Ethylhexanol.

In einer bevorzugten Ausführungsform des Verfahrens wird die Umsetzung in Substanz, also ohne Lösungsmittel durchgeführt.

Es können aber auch untergeordnete Mengen an Wasser anwesend sein, beispielsweise bis zu 20 Gew%, bevorzugt bis zu 15, besonders bevorzugt bis zu 10 und ganz besonders bevorzugt bis zu 5 Gew% bzgl. des Salzes des Lysin.

Das bei der Reaktion freiwerdende Wasser kann destillativ, gegebenenfalls unter Überleitung eines unter den Reaktionsbedingungen inerten Gases über die flüssigen Phase, unter Durchleitung eines unter den Reaktionsbedingungen inerten Gases durch die flüssige Phase, gegebenenfalls bei vermindertem Druck, abgetrennt und so aus dem Reaktionsgleichgewicht entfernt werden. Dadurch wird auch die Umsetzung beschleunigt.

Unter den Reaktionsbedingungen inerte Gase können beispielsweise sein Edelgase, wie beispielsweise Helium oder Argon, Stickstoff, Kohlenstoffmonoxid oder Kohlenstoffdioxid.

Zur Beschleunigung der Reaktion werden Katalysatoren (F) oder Katalysatorgemische zugegeben.

Geeignete Katalysatoren sind solche Verbindungen, die eine Ester- oder Amidbildung katalysieren, und ausgewählt aus der Gruppe der

- -- (F1) tertiären Amine und Amidine,
- -- (F2) basischen Alkalimetall-, Erdalkalimetall- oder quartären Ammoniumsalze und
- -- (F3) Alkanolate, Alkanoate, Chelate oder metallorganische Verbindungen der Metalle der Gruppen IIIA bis VIIIA oder IB bis VB im Periodensystem der Elemente.

Tertiäre Amine und Amidine (F1) sind solche, die an den Aminogruppen keine freien Wasserstoffatome aufweisen sondern die Stickstoffatome über drei Bindungen ausschließlich mit Kohlenstoffatomen verbunden sind. Bevorzugt sind solche tertiären Amine und Amidine, die einen $pK_B$-Wert von mehr als 8,9 aufweisen, besonders bevorzugt von mehr als 10,3. Ganz besonders bevorzugt weisen die tertiären Amine und Amidine bei der Reaktionstemperatur nur eine geringe Flüchtigkeit auf, insbesondere einen Siedepunkt oberhalb der Reaktionstemperatur.

Beispiele für tertiäre Amine sind Trioctylamin, Tridodecylamin, Tribenzylamin, N,N,N',N'-Tetramethylethylendiamin, 1-Methylpyrrol, Pyridin, 4-Dimethylaminopyridin, Picolin, N,N'-Dimethylpiperazin, N-Methylmorpholin, N-Methylpiperidin, N-Ethylpiperidin, N,N-Dimethylanilin, N,N-Dimethylbenzylamin, 1,4-Diazabicyclo[2.2.2]octan, 1,5-Diazabicyclo[4.3.0]non-5-en oder 1,8-Diazabicyclo[5.4.0]undec-7-en. Beispiele für Amidine sind Imidazole, wie N-Methylimidazol, Imidazol, 1-Methylimidazol, 2-Methylimidazol oder 1,2-Dimethylimidazol.

Bei den basischen Alkalimetall-, Erdalkalimetall- oder quartären Ammoniumsalzen (F2) handelt es sich um Hydroxide, Oxide, Carbonate, Hydrogencarbonate, $C_1$-$C_1$o-Alkanolate oder $C_1$-$C_{10}$-Alkanoate mit Kationen aus der Reihe der Alkalimetalle oder Erdalkalimetalle oder quartären Ammoniumionen.

Alkalimetalle sind bevorzugt Li, Na, K oder Cs, besonders bevorzugt Na und K. Erdalkalimetalle sind bevorzugt Mg und Ca. Quartäre Ammoniumionen können 4 bis 32 Kohlenstoffatome aufweisen und substituiert sein mit Alkyl, Cycloalkyl, Aryl oder Arylalkyl, bevorzugt mit Alkyl oder Arylalkyl und besonders bevorzugt mit Alkyl.

Bevorzugte $C_1$-$C_{10}$-Alkanolate sind $C_1$-$C_4$-Alkanolate, besonders bevorzugt sind Methanolat, Ethanolat, iso-Propanolat und n-Butanolat, ganz besonders bevorzugt sind Methanolat und Ethanolat und insbesondere Methanolat.

Bevorzugte $C_1$-$C_{10}$-Alkanoate sind $C_1$-$C_4$-Alkanoate, besonders bevorzugt ist Acetat.

Bevorzugte Verbindungen (F2) sind Lithium-, Natrium-, Kalium- oder Cäsiumhydroxid, Lithium-, Natrium-, Kalium- oder Cäsiumcarbonat, Lithium-, Natrium-, Kalium- oder Cäsiumacetat, besonders bevorzugt sind Natrium- oder Kaliumhydroxid. Verbindungen (F3) sind Alkanolate, Alkanoate, Chelate oder metallorganische Verbindungen der Metalle der Gruppen IIIA bis VIIIA oder IB bis VB im Periodensystem der Elemente.

Alkanolate sind beispielsweise $C_1$-$C_{10}$-Alkanolate, bevorzugt $C_1$-$C_4$-Alkanolate, besonders bevorzugt sind Methanolat, Ethanolat, iso-Propanolat und n-Butanolat, ganz besonders bevorzugt sind Methanolat und Ethanolat und insbesondere Methanolat.

Alkanoate sind beispielsweise $C_1$-$C_{20}$-Alkanoate, bevorzugt sind $C_1$-$C_4$-Alkanoate, besonders bevorzugt ist Acetat.

Chelate sind dabei cyclische Verbindungen, bei denen Metalle und Gruppierungen mit einsamen Elektronenpaaren einen Ring bilden. Bevorzugter Chelatbildner ist Acetylacetonat.

Metallorganische Verbindungen sind solche mit einer direkten Metall-Kohlenstoff-Bindung.

Bevorzugt Metalle sind Bor, Aluminium, Zinn, Zink, Titan, Antomin, Zirkon oder Wismut.

Bevorzugte Verbindungen (F3) sind Titantetrabutanolat, Titantetraisopropanolat, Zirkonacetylacetonat, Zirkontetrabutanolat, Zinn(II)-n-octanoat, Zinn-(II)-2-ethylhexanoat, Zinn-(II)-laurat, Dibutyl-zinnoxid, Dibutylzinndichlorid, Dibutylzinndiacetat, Dibutylzinn-dilaurat, Dibutylzinndimaleat, Dioctylzinndiacetat, Antimontriethanolat oder Boronsäurederivate, beispielsweise Pyridinboronsäure.

Bevorzugte Katalysatoren sind (F1) und (F2), besonders bevorzugt sind Verbindungen (F2).

Die Verbindungen (A) bis (E) können für die Reaktion in folgenden Verhältnissen miteinander umgesetzt werden:

(B) bis zu 100 mol% bezogen auf (A), bevorzugt 0 bis 75 mol%, besonders bevorzugt 0 - 50 mol%, ganz besonders bevorzugt 0 - 25 mol%, insbesondere 0 - 15 mol% und speziell 0 mol%,

(C) 0 bis 50 mol% bezogen auf (A), bevorzugt 0 bis 30 mol%, besonders bevorzugt 0 - 25 mol%, ganz besonders bevorzugt 0 - 10 mol%, insbesondere 0 - 5 mol% und speziell 0 mol%,

(D) 0 bis 50 mol% bezogen auf (A), bevorzugt 0 bis 30 mol%, besonders bevorzugt 0 - 25 mol%, ganz besonders bevorzugt 0 - 10 mol%, insbesondere 0 - 5 mol% und speziell 0 mol%,

(E) 0 - 200 Gew% bzgl. der Summe der Komponenten (A) bis (D), bevorzugt 0 - 100 Gew%, besonders bevorzugt 0 - 75 Gew%, ganz besonders bevorzugt 0 - 50 Gew%, insbesondere 0 - 25 Gew% und speziell 0 Gew% und

(F1) oder

(F2) bis zu 110 mol%, bevorzugt bis zu 105 mol%, besonders bevorzugt bis zu 100 mol% und in der Regel mindestens 80 mol% bzgl. der Säure, die das Salz mit Lysin bildet, bzw.

(F3) 0,1 bis 20 mol% bzgl. der Summe der Komponenten (A) bis (D) und bevorzugt 0,1 bis 15 mol%.

Die nach dem Verfahren hergestellten hochfunktionellen hochverzweigten Polypeptide sind nach der Umsetzung, also ohne weitere Modifikation, mit Amino- und/oder mit Carboxylgruppen terminiert. Sie lösen sich gut in polaren Lösemitteln, zum Beispiel in Wasser, Alkoholen, wie Methanol, in modifizierter Form dann auch in Ethanol, Butanol, Alkohol/Wasser-Mischungen, Dimethylformamid, Dimethylacetamid, N-Methylpyrrolidon, Chloroform, Ethylencarbonat oder Propylencarbonat.

Unter einem hochfunktionellen Polypeptid im Sinne der Erfindung wird ein Produkt verstanden, das mindestens drei, bevorzugt mindestens sechs, insbesondere mindestens zehn funktionelle Gruppen aufweist. Die Anzahl der funktionellen Gruppen ist prinzipiell nach oben nicht beschränkt, jedoch können Produkte mit einer sehr hohen Anzahl von funktionellen Gruppen unerwünschte Eigenschaften, beispielsweise eine hohe Viskosität oder eine schlechte Löslichkeit, aufweisen. Die hochfunktionellen Polypeptide der vorliegenden Erfindung weisen im Allgemeinen nicht mehr als 200 funktionelle Gruppen, bevorzugt nicht mehr als 100 funktionelle Gruppen auf. Unter funktionellen Gruppen sind hier primäre, sekundäre oder tertiäre Aminogruppen oder Carboxylgruppen zu verstehen. Daneben kann das hochfunktionelle hochverzweigte Polypeptid weitere funktionelle Gruppen aufweisen, die nicht am Aufbau des hochverzweigten Polymers teilnehmen (siehe unten). Diese weiteren funktionellen Gruppen können durch Di- oder Polyamine beziehungsweise Di- oder Polycarbonsäuren eingeführt werden, welche neben primären und sekundären Aminogruppen beziehungsweise Säuregruppen noch weitere funktionelle Gruppen aufweisen.

In einer weiteren bevorzugten Ausführungsform können die erfindungsgemäßen Polypeptide weitere funktionelle Gruppen enthalten. Die Funktionalisierung kann dabei während der Umsetzung, also während der den Molekulargewichtsaufbau bewirkenden Polykondensationsreaktion, oder aber nach Beendigung der Polykondensationsreaktion durch nachträgliche Funktionalisierung der erhaltenen Polypeptide erfolgen.

Gibt man vor oder während des Molekulargewichtsaufbaus Komponenten zu, die neben Amino- und/oder Carboxylgruppen weitere funktionelle Gruppen aufweisen, so erhält man ein Polypeptid mit statistisch verteilten weiteren, das heißt von den Amino- oder Carboxylgruppen verschiedenen funktionellen Gruppen.

Beispielsweise können vor oder während der Polykondensation Komponenten (G) zugegeben werden, die entweder primäre oder sekundäre Aminogruppen oder Carboxylgruppen, Hydroxylgruppen, Mercaptogruppen, tertiäre Aminogruppen, Ethergruppen, Harnstoffgruppen, Sulfonsäuregruppen, Phosphonsäuregruppen, Silangruppen, Siloxangruppen, Arylreste oder kurz- oder langkettige, gegebenenfalls auch teilweise fluorierte oder perfluorierte, lineare oder verzweigte Alkylreste aufweisen.

Hydroxylgruppen aufweisende Komponenten (G), die zur Funktionalisierung zugesetzt werden können, umfassen beispielsweise Ethanolamin, N-Methylethanolamin, Propanolamin, Isopropanolamin, Butanolamin, 2-Amino-1-butanol, 2-(Butylamino)ethanol, 2-(Cyclohexylamino)ethanol, 2-(2'-Aminoethoxy)ethanol oder höhere Alkoxylierungsprodukte des Ammoniaks, 4-Hydroxypiperidin, 1-Hydroxyethylpiperazin, Diethanolamin, Dipropanolamin, Diisopropanolamin, Tris(hydroxymethyl)aminomethan oder Tris(hydroxyethyl)aminomethan sowie Dimethylolpropionsäure, Dimethylolbuttersäure, Hydroxypivalinsäure, Milchsäure oder Glykolsäure.

Mercaptogruppen enthaltende Komponenten, die zur Funktionalisierung zugesetzt werden können, umfassen beispielsweise Cysteamin, Mercaptoessigsäure oder Mercaptoethanol. Mit tertiären Aminogruppen lassen sich die hochverzweigten Polypeptide zum Beispiel durch Mitverwendung von Di(aminoethyl)methylamin, Di(aminopropyl)-methylamin oder N,N-Dimethylethylendiamin funktionalisieren. Mit Ethergruppen lassen sich die hochverzweigten Polypeptide durch Mitverwendung von Amin-terminierten Polyetherolen (sogenannten Jeffaminen) oder mit Polyether-Carbonsäuren funktionalisieren. Mit Sulfonsäure- oder Phosphonsäuregruppen lassen sich die hochverzweigten Polypeptide zum Beispiel durch Mitverwendung von Aminosulfonsäuren oder Aminophosphonsäuren funktionalisieren. Mit Silizium enthaltenden Gruppen lassen sich die hochverzweigten Polypeptide durch Mitverwendung von Hexamethyldisilazan, N-(3-Trimethylsilylethyl)ethylendiamin, 3-Aminopropyltrimethylsiloxan, 3-Aminopropyltriethylsiloxan, 3-Isocyanatopropyltrimethylsiloxan oder 3-Isocyanatopropyltriethylsiloxan funktionalisieren. Mit langkettigen Alkylresten lassen sich die hochverzweigten Polypeptide durch Mitverwendung von Alkylaminen, Alkylcarbonsäuren, Alkylsulfonsäuren oder Alkylisocyanaten funktionalisieren.

Weiterhin lassen sich die Polypeptide auch durch Einsatz geringer Mengen an Monomeren (H) funktionalisieren, die keine Aminogruppen und keine Carboxylgruppen aufweisen, sondern von Aminogruppen oder Carboxylgruppen verschiedene funktionelle Gruppen aufweisen, die mit Amino- oder Carboxylgruppen reagieren. Genannt seien hier beispielsweise di-, tri- oder höherfunktionelle Alkohole, die über Esterfunktionen in das Polypeptid eingebaut werden können. So lassen sich zum Beispiel hydrophobe Eigenschaften durch Zusatz langkettiger Alkandiole erzielen, während Polyethylenoxiddiole oder -triole hydrophile Eigenschaften im Polypeptid erzeugen.

Die genannten, von Amin- oder Carbonsäuregruppen verschiedenen funktionellen Gruppen, die vor oder während der Polykondensation eingeführt werden, werden im Allgemeinen in Mengen von 0,1 bis 80 mol.-%, bevorzugt in Mengen von 1 bis 50 mol.-%, bezogen auf die Summe der Amino- und Carbonsäuregruppen, eingeführt.

Ein weiterer Gegenstand der vorliegenden Erfindung ist die Verwendung von modifizierten Polylysinen, in denen die zugänglichen Amino-und/oder Carboxylgruppen zumindest teilweise weiter modifiziert sind, also mit Reagenzien umgesetzt sind, die die Eigenschaften des so modifizierten Polylysins verändern. Eigenschaften sind dabei beispielsweise Löslichkeit, Dispergierbarkeit, Hydrophilie, Hydrophobie und Rheologie.

Die Modifizierung der Polylysine erfolgt bevorzugt mit den Polylysinen, wie sie oben beschrieben sind, deren Herstellung auf der Umsetzung eines Salzes von Lysin mit einer Säure beruht. Denkbar ist aber auch die Modifizierung von beliebig erhaltenen Polylysinen, die beispielsweise hergestellt worden sind durch Polymerisation oder Copolymerisation von anderen lysinhaltigen Edukten als (A), beispielsweise freier Lysin-Base.

Die für eine derartige Modifizierung einsetzbaren Polylysine haben ein gewichtsmittleres Molekulargewicht $M_w$ von mehr als 7.500 Da, mehr als 10.000 Da, mehr als 15.000 Da oder sogar mehr als 20.000 Da.

Eine obere Grenze für das gewichtsmittlere Molekulargewicht $M_w$ ist bis zu 750.000 Da, bevorzugt bis zu 600.000 Da, besonders bevorzugt bis 500.000 Da, ganz besonders bevorzugt bis zu 400.000 Da und insbesondere bis zu 300.000 Da.

Die einsetzbaren Polylysine können beispielsweise einen Gehalt an primären, sekundären oder tertiären, freien oder protonierten Aminogruppen, berechnet als $NH_2$, von 1 bis 21,9 Gew%, bevorzugt 3 bis 18 Gew% aufweisen.

Die einsetzbaren Polylysine können beispielsweise eine Gehalt an freien oder deprotonierten Säuregruppen, berechnet als COOH, von 0 bis 30 Gew% und bevorzugt 0 bis 15 Gew% aufweisen.

Eine nachträgliche Funktionalisierung von Aminogruppen enthaltenden hochfunktionellen hochverzweigten Polypeptiden kann zum Beispiel erreicht werden durch Zugabe von Säuregruppen, Isocyanatgruppen, Ketogruppen oder Aldehydgruppen oder aktivierte Doppelbindungen enthaltenden Molekülen ohne Amino- oder Carboxylgruppen (I), zum Beispiel acrylische Doppelbindungen, enthaltenden Molekülen. Beispielsweise lassen sich Säuregruppen enthaltende Polypeptide durch Umsetzung mit Acrylsäure oder Maleinsäure und deren Derivaten, beispielsweise Ester, mit gegebenenfalls anschliessender Hydrolyse erhalten.

Weiterhin können Aminogruppen enthaltende hochfunktionelle Polypeptide durch Umsetzung mit Alkylenoxiden (J1), zum Beispiel Ethylenoxid, Propylenoxid oder Butylenoxid, in hochfunktionelle Polypeptid-Polyole überführt werden.

Eine weitere Möglichkeit zur Funktionalisierung der Aminogruppen in den hochfunktionellen Polypeptiden besteht in der zumindest teilweisen Umsetzung der Aminogruppen mit Lactonen und/oder Lactamen unter Bildung von auf diesen Aminogruppen gestarteten Polyesterketten mit endständiger Hydroxygruppe. Beispielhafte Lactame sind ε-Caprolactam, δ-Valerolactam, γ-Butyrolactam, N-Methylcaprolactam und N-Methylpyrrolidon. Beispielhafte Lactone sind ε-Caprolacton, δ-Valerolacton und γ-Butyrolacton.

Eine weitere Möglichkeit der Herstellung von Polypeptid/Polyether-Verbindungen besteht in der Umsetzung der Polypeptide mit mono-, di- oder höherfunktionellen Aminogruppen- oder Säuregruppen-terminierten Polyalkylenoxyden (J2), vorzugsweise Polyethylenoxiden, Polypropylenoxiden oder Polyethylenpropylenoxiden.

Durch Salzbildung mit Protonensäuren oder durch Quaternisierung der Aminofunktionen mit Alkylierungsreagenzien (K), wie Methylhalogeniden, Alkyltosylaten oder Dialkylsulfaten, können die hochfunktionellen, hochverzweigten Polypeptide wasserlöslich oder wasserdispergierbar eingestellt werden.

Die Salzbildung kann auch durchgeführt werden, indem die Aminogruppen der erfindungsgemäßen hyperverzweigten Polymere stöchiometrisch oder unterstöchiometrisch mit solchen sauren Komponenten oder deren Salzen vermischt oder umgesetzt werden, die langkettige lineare oder verzweigte Alkylreste, gegebenenfalls substituierte Cycloalkylreste oder gegebenenfalls substituierte Arylreste aufweisen und die allgemein als Seifen oder Tenside bekannt sind.

Derartige saure Komponenten können bevorzugt mindestens eine, besonders bevorzugt genau eine Carboxyl-, Sulfonsäure-, Sulfat- oder Phosphonsäuregruppen aufweisen.

Beispielsweise können die hyperverzweigten Polymere mit Alkyl- oder Alkenylcarbonsäuren, wie zum Beispiel Octansäure, Nonansäure, Decansäure, Dodecansäure, Hexadecansäure, Stearinsäure, Ölsäure, Linolsäure, Linolensäure oder deren Li-, Na-, K-, Cs-, Ca- oder Ammoniumsalzen, mit Alkylsulfonsäuren, zum Beispiel Octansulfonsäure, Dodecansulfonsäure, Stearylsulfonsäure oder Oleylsulfonsäure, oder deren Li-, Na- , K-, Cs-, Ca- oder Ammoniumsalzen, mit Camphersulfonsäure, Cyclododecylsulfonsäure, p-Toluolsulfonsäure, Benzolsulfonsäure, 4-Hexylbenzolsulfonat, 4-Octylbenzolsulfonat, 4-Decylbenzolsulfonat oder 4-Dodecylbenzolsulfonat oder deren Li-, Na-, K-, Cs-, Ca- oder Ammoniumsalzen, mit Alkylsulfaten, zum Beispiel mit n-Alkylsulfaten oder sekundären Alkylsulfaten umgesetzt werden. Dabei entstehen ionische Polylysin-Tensid-Komplexe, die zum Beispiel flüssigkristalline Eigenschaften aufweisen oder als polymere Ionische Flüssigkeiten wirken können.

Die Alkyl-, Cycloalkyl-, oder Arylreste können dabei bis zu 20 Kohlenstoffatome aufweisen, bevorzugt 6 bis 20, besonders bevorzugt 7 bis 20.

Um eine Hydrophobierung zu erreichen, können Amin-terminierte hochfunktionelle hochverzweigte Polypeptide mit gesättigten oder ungesättigten langkettigen Carbonsäuren (L), deren gegenüber Amin-Gruppen reaktiven Derivaten oder auch mit aliphatischen oder aromatischen Isocyanaten, Trialkylsilylhalogeniden, teilweise oder vollständig fluorierten Alkoholen, Alkylhalogeniden, Carbonsäuren oder Aminen umgesetzt werden.

Mit Carbonsäuregruppen terminierte Polypeptide können durch Umsetzung mit langkettigen Alkylaminen oder langkettigen aliphatischen Monoalkoholen hydrophobiert werden.

Um eine nichtionische Hydrophilierung zu erreichen, können Amin-terminierte hochfunktionelle hochverzweigte Polypeptide mit aliphatischen oder aromatischen Isocyanaten umgesetzt werden, die weiterhin Polyethylenglykolketten enthalten.

Mit Carbonsäuregruppen terminierte Polypeptide können durch Umsetzung mit langkettigen, vorzugsweise monofunktionellen Polyethylenglykolen oder Polyethylenglykolaminen (Jeffaminen) nichtionisch hydrophiliert werden.

Um einen amphiphilen Charakter zu erreichen, können die hochfunktionellen, hochverzweigten Polypeptide auch mit hydrophoben und hydrophilen Agenzien gleichzeitig modifiziert werden, zum Beispiel mit mono-, di- oder höherfunktionellen langkettigen aliphatischen Carbonsäuren, Alkoholen, Aminen oder Isocyanaten und gleichzeitig mit mono-, di- oder höherfunktionellen Polyethylenglykolketten aufweisenden Alkoholen, Aminen, Säuren oder Isocyanaten.

Zur Reinigung, speziell auch zur Abtrennung der bei der Herstellung entstehenden anorganischen Salze, lassen sich die erfindungsgemäßen Polymere zum Beispiel in polaren oder unpolaren Lösemitteln lösen, wobei die Salze nicht in Lösung gehen und durch Filtration von dem Polymeren getrennt werden können. Beispielhaft sei hier das Lösen des unmodifizierten Polylysins in Ethanol genannt, wobei sich das bei der Reaktion entstandene Kaliumchlorid als Bodensatz niederschlug und durch Abfiltrieren von der Polymerlösung getrennt werden konnte.

Gegenstand der vorliegenden Erfindung ist die Verwendung der mit mindestens einer Säuregruppe enthaltenden Verbindung modifizierten Polypeptide als Haftvermittler und Thixotropiermittel, Löslichkeitsvermittler (Solubilisatoren), als Phasentransferreagenz für in Wasser unlösliche Chemikalien, als Phasentransferreagenz für in Wasser lösliche Chemikalien, oder als Surface-Modifier. Diese modifizierten Polylysine können als Komponenten zur Herstellung von Druckfarben, Lacken, Überzügen, Klebstoffen, Dichtmassen, Korrosionsschutzmitteln, Gießelastomeren und Schaumstoffen eingesetzt werden.

Beispiele:

Beispiel 1: Kondensationsprodukt aus L-Lysin*1 HCl, Reaktion bei 150 °C unter NaOH-Zugabe ohne Vakuum

L-Lysin*HCl (11 g, 60 mmol) und festes NaOH (2,4 g, 60 mmol) wurden in einer Reibschale verrieben und die Mischung anschließend in einem Schlenkrohr auf 150 °C erhitzt. Während der Reaktion wurden nach 14, 24, 38 und 48 Stunden Proben entnommen, in Wasser gelöst, die Lösung filtert, und die Molekulargewichte per GPC-Analytik bestimmt. Die GPC-Analytik erfolgte an unbehandelten Proben, die direkt der Reaktionsmischung entnommen wurden, mittels Säulenkombination aus OHpak SB-803 HQ und SB-804 HQ (Fa. Shodex) in wässriger Lösung unter Zugabe von 0,1 mol/l Natriumhydrogencarbonat bei 30°C mit einer Flussrate von 0,5 ml/min und Polyethylenoxid als Standard. Zur Detektion wurde ein UV-Detektor eingesetzt, der bei einer Wellenlänge von 230 nm arbeitete.

Tabelle 1: Polykondensation von L-Lysin*HCl bei 150°C unter Zugabe von NaOH

| Reaktionszeit | Molekulargewicht (Mw) | Polydispersität |
|---|---|---|
| 14 Stunden | 2500 g/mol | 2,2 |
| 24 Stunden | 3400 g/mol | 2,4 |
| 38 Stunden | 14600 g/mol | 5 |
| 48 Stunden | 28100 g/mol | 9,3 |

Beispiel 2: Kondensationsprodukt aus L-Lysin*HCl, Reaktion bei 150 °C unter KOH-Zugabe ohne Vakuum

L-Lysin*HCl (11 g, 60 mmol) und KOH (3,3 g, 60 mmol) wurden in einer Reibschale verrieben und die Mischung in einem Schlenkrohr auf 150 °C erhitzt. Während der Reaktion wurden nach 14, 24, 38 und 48 Stunden Proben entnommen, in Wasser gelöst, filtriert und die Molekulargewichte wie unter Beispiel 1 beschrieben bestimmt (siehe Tabelle 2).

Tabelle 2: Polykondensation von L-Lysin*HCl bei 150°C unter Zugabe von KOH

| Reaktionszeit | Molekulargewicht (Mw) | Polydispersität |
|---|---|---|
| 14 Stunden | 3300 g/Mol | 2,8 |
| 24 Stunden | 9900 g/Mol | 4,9 |
| 38 Stunden | 36100 g/Mol | 11,3 |
| 48 Stunden | 283700 g/Mol | 61,8 |

Beispiel 3: Kondensationsprodukt aus L-Lysin*HCl, Reaktion bei 150 °C unter NaOH- und Zirkon(IV)-butanolat-Zugabe ohne Vakuum

L-Lysin*HCl (11 g, 60 mmol) und NaOH (2,4 g, 60 mmol) wurden in einer Reibschale verrieben und die Mischung nach Zugabe von 1 ml Zirkon(IV)butanolat ($Zr(OBu)_4$) in einem Schlenkrohr auf 150 °C erhitzt. Während der Reaktion wurden nach 14, 24, 38 und 48 Stunden Proben entnommen, in Wasser gelöst, filtriert und die Molekulargewichte per GPC wie unter Beispiel 1 beschrieben bestimmt (siehe Tabelle 3).

Tabelle 3: Polykondensation von L-Lysin*HCl bei 150°C unter Zugabe von NaOH und Zirkon(IV)butanolat

| Reaktionszeit | Molekulargewicht (Mw) | Polydispersität |
|---|---|---|
| 14 Stunden | 3100 g/Mol | 2,0 |
| 24 Stunden | 7700 g/Mol | 2,8 |

(fortgesetzt)

| Reaktionszeit | Molekulargewicht (Mw) | Polydispersität |
|---|---|---|
| 38 Stunden | 25700 g/Mol | 5,5 |
| 48 Stunden | 57300 g/Mol | 10,4 |

Beispiel 4: Kondensationsprodukt aus L-Lysin*HCl, Reaktion bei 150 °C unter KOH- und Zirkon(IV)-butanolat-Zugabe ohne Vakuum

L-Lysin*HCl (11 g, 60 mmol) und KOH (3,3 g, 60 mmol) wurden in einer Reibschale verrieben und die Mischung nach Zugabe von 1 ml Zirkon(IV)butanolat ($Zr(OBu)_4$) in einem Schlenkrohr auf 150 °C erhitzt. Während der Reaktion wurden nach 14, 24, 38 und 48 Stunden Proben entnommen, in Wasser gelöst, filtriert und die Molekulargewichte per GPC wie unter Beispiel 1 beschrieben bestimmt (siehe Tabelle 4).

Tabelle 4: Polykondensation von L-Lysin*HCl bei 150°C unter Zugabe von KOH und Zirkon(IV)butanolat

| Reaktionszeit | Molekulargewicht (Mw) | Polydispersität |
|---|---|---|
| 14 Stunden | 4900 g/Mol | 2,5 |
| 24 Stunden | 19400 g/Mol | 4,6 |
| 38 Stunden | 139000 g/Mol | 23 |
| 48 Stunden | 510000 g/Mol | 107 |

Beispiel 5: Kondensationsprodukt aus L-Lysin*HCl, Reaktion bei 150 °C unter NaOH- und Dibutylzinn-dilaurat-Zugabe ohne Vakuum

L-Lysin*HCl (11 g, 60 mmol) und NaOH (2,4 g, 60 mmol) wurden in einer Reibschale verrieben und die Mischung nach Zugabe von 1 ml Dibutylzinn-dilaurat in einem Schlenkrohr auf 150 °C erhitzt. Während der Reaktion wurden nach 14, 24, 38 und 48 Stunden Proben entnommen, in Wasser gelöst, filtriert und die Molekulargewichte per GPC wie unter Beispiel 1 beschrieben bestimmt (siehe Tabelle 5).

Tabelle 5: Polykondensation von L-Lysin*HCl bei 150°C unter Zugabe von NaOH und Dibutvlzinn-dilaurat

| Reaktionszeit | Molekulargewicht (Mw) | Polydispersität |
|---|---|---|
| 14 Stunden | 2300 g/Mol | 2,5 |
| 24 Stunden | 5300 g/Mol | 4,1 |
| 38 Stunden | 37000 g/Mol | 21,2 |
| 48 Stunden | 49400 g/Mol | 27,9 |

Beispiel 6: Kondensationsprodukt aus L-Lysin*HCl, Reaktion bei 150 °C unter NaOH- und Triphenylphosphit-Zugabe ohne Vakuum

L-Lysin*HCl (11 g, 60 mmol) und NaOH (2,4 g, 60 mmol) wurden in einer Reibschale verrieben und die Mischung nach Zugabe von 1 ml Triphenylphosphit in einem Schlenkrohr auf 150 °C erhitzt. Während der Reaktion wurden nach 14, 24, 38 und 48 Stunden Proben entnommen, in Wasser gelöst, filtriert und die Molekulargewichte per GPC wie unter Beispiel 1 beschrieben bestimmt (siehe Tabelle 6).

Tabelle 6: Polykondensation von L-Lysin*HCl bei 150°C unter Zugabe von NaOH und Triphenylphosphit

| Reaktionszeit | Molekulargewicht (Mw) | Polydispersität |
|---|---|---|
| 14 Stunden | 3200 g/Mol | 3,2 |
| 24 Stunden | 6400 g/Mol | 4,6 |
| 38 Stunden | 14000 g/Mol | 8,7 |

(fortgesetzt)

| Reaktionszeit | Molekulargewicht (Mw) | Polydispersität |
|---|---|---|
| 48 Stunden | 18400 g/Mol | 12,7 |

Beispiel 7: Kondensationsprodukt aus L-Lysin*HCl, Reaktion bei 180 °C unter NaOH-Zugabe ohne Vakuum

L-Lysin*HCl (5,5 g, 30 mmol) und NaOH (1,2 g, 30 mmol) wurden in einer Reibschale verrieben und die Mischung in einem Schlenkrohr auf 180 °C erhitzt. Nach 24 Stunden wurde auf Raumtemperatur abgekühlt, die viskose Schmelze in Wasser gelöst und filtriert. Das Molekulargewicht Mw des Polymers, bestimmt per GPC gemäß Beispiel 1, betrug 20600 g/Mol, die Polydispersität 4,9.

Beispiel 8: Kondensationsprodukt aus L-Lysin*HCl, Reaktion bei 150 °C mit NaOH unter Vakuum

L-Lysin*HCl (11 g, 60 mmol) und NaOH (2,4 g, 60 mmol) wurden in einer Reibschale verrieben und die Mischung in einem Schlenkrohr unter Vakuum auf 150 °C erhitzt. Während der Reaktion wurden nach 14, 24, 38 und 48 Stunden Proben entnommen, in Wasser gelöst, filtriert und die Molekulargewichte per GPC wie unter Beispiel 1 beschrieben bestimmt (siehe Tabelle 8).

Tabelle 8: Polykondensation von L-Lysin*HCl bei 150°C unter Zugabe von NaOH unter Vakuum

| Reaktionszeit | Molekulargewicht (Mw) | Polydispersität |
|---|---|---|
| 14 Stunden | 5800 g/mol | 3,3 |
| 24 Stunden | 29300 g/mol | 8,4 |
| 38 Stunden | 122800 g/mol | 24,7 |
| 48 Stunden | 503600 g/mol | 133,1 |

Beispiel 9: Kondensationsprodukt aus L-Lysin*HCl, Reaktion bei 150 °C mit NaOH und Dibutylzinn-dilaurat unter Vakuum

In einen 41-Vierhalskolben, ausgestattet mit Rührer, Innenthermometer, Gaseinleitrohr und absteigendem Kühler mit Vakuumanschluß und Auffanggefäß wurden 1000 g L-Lysin-Hydrochlorid, 218g festes Natriumhydroxid, 100g Wasser und 0,3g Dibutylzinn-dilaurat gegeben und die Mischung unter Rühren auf eine Innentemperatur von 150°C aufgeheizt. Nach 5 Stunden Reaktionsdauer wurde Wasser unter vermindertem Druck (200 mbar) abdestilliert, wobei nach Übergang der größten Menge an Wasser die Temperatur langsam auf 180°C erhöht und der Druck auf 10 mbar reduziert wurde. Nach 8 Stunden waren 240g Wasser als Destillat aufgefangen.
Das hochviskose Polymer wurde heiß ausgetragen, auf ein Kühlblech gegossen und anschließend in einem Mörser kleingemahlen.
Die Bestimmung der Glastemperatur ergab eine Tg von 36,8 °C.
Zur Bestimmung der Molekulargewichtsverteilung wurde das feste Produkt in Wasser gelöst, die Lösung filtriert, und per GPC nach dem unter Beispiel 1 genannten Verfahren vermessen. Das gewichtsmittlere Molekulargewicht Mw betrug 15.000 g/mol, die Polydispersität betrug 5,0.

Vergleichsbeispiel 10, Kondensation von L-Lysin ohne Katalysator (analog Harada, Bull. Chem. Soc. Japan 1959, 32, 1007-1008)

L-Lysin wurde in einem Schlenkrohr auf 150 °C erhitzt. Nach 48 Stunden wurde das Produkt in Wasser gelöst, filtriert und das Molekulargewicht wie unter Beispiel 1 beschrieben bestimmt. Das gewichtsmittlere Molekulargewicht Mw betrug 2400 g/mol, die Polydispersität lag bei 2,2.

Vergleichsbeispiel 11, Vergleichsbeispiel nach Lit. Rohlfing et al. Arch. Biochem. Biophys. 130, 441 (1969))

L-Lysin wurde in einem Schlenkrohr auf 192 °C unter Stickstoffatmosphäre erhitzt. Nach 3 Stunden wurde ein Molekulargewicht mit Mw = 7400 (Polydispersität = 3,9), nach 8 Stunden ein Mw = 15900 (Polydispersität =10) bestimmt. Danach setzte Vernetzung ein, nach 24 Stunden waren bereits 70% des eingesetzten Materials in Wasser unlöslich.

Vergleichsbeispiel 12, Vergleichsbeispiel nach Lit. Fox et al. (BioSystems 1976, 8, 40-44, Beispiel S. 40, rechte Spalte oben)

L-Lysin*HCl wurde zusammen mit Orthophosphorsäure (1 ml pro 0,64g Lysin-Hydrochlorid) in einem Schlenkrohr auf 195 °C erhitzt. Nach 10 Stunden wurde das Reaktionsprodukt in Wasser gelöst, die Säure mit NaOH neutralisiert und das Produkt per GPC analysiert: gewichtsmittleres Molekulargewicht Mw = 1100, Polydispersität = 3,1.

Vergleichsbeispiel 13, Vergleichsbeispiel analog Lit. Fox et al. (BioSystems 1976, 8, 40-44)

L-Lysin*HCl wurde zusammen mit Orthophosphorsäure (1 ml pro 3,5g Lysin-Hydrochlorid) in einem Schlenkrohr auf 195 °C erhitzt. Nach 10 Stunden wurde das Reaktionsprodukt in Wasser gelöst, die Säure mittels NaOH neutralisiert und das Produkt per GPC analysiert: gewichtsmittleres Molekulargewicht Mw = 4300, Polydispersität = 1,07.

Beispiel 14: Hydrophob-Modifikation von Polylysin

In einen 11-Vierhalskolben, ausgestattet mit Rührer, Innenthermometer, Gaseinleitrohr und absteigendem Kühler mit Vakuumanschluß und Auffanggefäß wurden 100 g L-Lysin-Hydrochlorid, 21,8g festes Natriumhydroxid und 20g Wasser gegeben und die Mischung unter Rühren auf eine Innentemperatur von 160°C aufgeheizt. Nach 5 Stunden Reaktionsdauer wurde Wasser unter vermindertem Druck (200 mbar) abdestilliert. Dann wurden 10g Stearinsäure zugegeben, die Temperatur auf 180°C erhöht und eine Stunde bei einem Druck von 80 mbar unter weiterer Wasserabscheidung reagieren gelassen. Das hochviskose Polymer wurde heiß ausgetragen, auf ein Kühlblech gegossen und anschließend in einem Mörser kleingemahlen.
Die Bestimmung der Glastemperatur ergab eine Tg von 29°C.
Zur Bestimmung der Molekulargewichtsverteilung wurde das feste Produkt in Wasser gelöst, die Lösung filtriert, und per GPC nach dem unter Beispiel 1 genannten Verfahren vermessen. Das gewichtsmittlere Molekulargewicht Mw betrug 7400 g/mol, die Polydispersität betrug 3,0.

Beispiel 15: Hydrophob-Modifikation von Polylysin

In einen 11-Vierhalskolben, ausgestattet mit Rührer, Innenthermometer, Gaseinleitrohr und absteigendem Kühler mit Vakuumanschluß und Auffanggefäß wurden 100 g L-Lysin-Hydrochlorid, 21,8g festes Natriumhydroxid und 20g Wasser gegeben und die Mischung unter Rühren auf eine Innentemperatur von 160°C aufgeheizt. Nach 5 Stunden Reaktionsdauer wurde Wasser unter vermindertem Druck (200 mbar) abdestilliert. Dann wurden 30g Stearinsäure zugegeben, die Temperatur auf 180°C erhöht und eine Stunde bei einem Druck von 80 mbar unter weiterer Wasserabscheidung reagieren gelassen. Das hochviskose Polymer wurde heiß ausgetragen, auf ein Kühlblech gegossen und anschließend in einem Mörser kleingemahlen.
Die Bestimmung der Glastemperatur ergab eine Tg von 36°C.
Zur Bestimmung der Molekulargewichtsverteilung wurde das feste Produkt in Wasser gelöst, die Lösung filtriert, und per GPC nach dem unter Beispiel 1 genannten Verfahren vermessen. Das gewichtsmittlere Molekulargewicht Mw betrug 24100 g/mol, die Polydispersität betrug 9,3.

Beispiel 16: Nachträgliche Modifizierung von Polylysin

1,5g festes Polylysin nach Beispiel 9 wurde mit 7,1 g Stearinsäure versetzt und die Mischung in einem Schlenkrohr auf 150°C erwärmt. Nach 6 Stunden wurde auf Raumtemperatur abgekühlt, das Gemisch in Tetrahydrofuran (THF) aufgelöst und die Lösung filtriert. Anschließend wurde das Polymer in Aceton ausgefällt, der Feststoff abfiltriert und im Vakuum bei 60°C getrocknet.
$^1$H-NMR (400MHz, CDCl$_3$): 4,45 (mb, 1 H, O=C-CH-NH-); 4,02 (m, 1 H, O=C-CH-NH-) 3,19 (m, 2H, -CH$_2$-NH); 2,31 (t, 2H, -CH$_2$-CH$_2$-COOH); 1,61 (q, 6 H, -CH$_2$-CH$_2$-COOH, CH$_2$-CH$_2$-NH); 1,26 (m, 30 H, -CH$_2$-CH$_2$-CH$_2$-); 0,86 (t, 3 H, -CH$_2$-CH$_3$).
IR: 3280*m*, 3076*w*, 2922*s*, 2852*m*, 1637*m*, 1533*m*, 1456*m*, 1377*w*, 1246*w*.

Beispiel 17: Nachträgliche Modifizierung von Polylysin

1,5g festes Polylysin nach Beispiel 9 wurde mit 6,7 g Ölsäure versetzt und die Mischung in einem Schlenkrohr auf 150°C erwärmt. Nach 6 Stunden wurde auf Raumtemperatur abgekühlt, das Gemisch in THF aufgelöst, die Lösung filtriert und THF am Rotationsverdampfer bei 60°C im Vakuum entfernt.
$^1$H-NMR (400MHz, CDCl$_3$): 5,33 (m, 2H, -CH=CH-); 4,47 (mb, 1H, O=C-CH-NH-); 3,75 (t, 1H, O=C-CH-NH-); 3,2 (m,

2H, -CH$_2$-NH); 2,32 (t, 2H, -CH$_2$-); 2,01 (d, 4 H, -CH$_2$-CH=CH-CH$_2$-); 1,85 (q, 2H, O=C-(NH)CH-CH$_2$-); 1,61 (m, 4 H, -CH$_2$-CH$_2$-CH$_2$-, CH$_2$-CH$_2$-NH); 1,28 (m, 24 H, CH$_2$-CH$_2$-CH$_2$-, -CH$_2$-CH$_2$-CH$_2$-NH); 0,85 (t, 3 H -CH$_2$-CH$_3$).
IR: 3295wb, 2926s, 2852m, 1709m, 1639m, 1548w, 1460w.

Beispiel 18: Nachträgliche Modifizierung von Polylysin

1,5g festes Polylysin nach Beispiel 9 wurde mit 5,5 g Polyethylenglykol-carbonsäure (mittleres Molekulargewicht 750g/mol) versetzt und die Mischung in einem Schlenkrohr auf 150°C erwärmt. Nach 6 Stunden wurde auf Raumtemperatur abgekühlt, das Gemisch in Wasser aufgenommen, die Lösung filtriert und mit einem Dialyseschlauch (MWCO 1000) von niedermolekularen Bestandteilen befreit. Anschließend wurde das Wasser über einen Gefriertrocknungsprozess entfernt.
$^1$H-NMR (400MHz, CDCl$_3$): 4,39 (m, 1 H, O=C-CH-NH-); 3,92 ((m, 1 H, O=C-CH-NH-); 3,7-3,2 (m, -O-CH$_2$-CH$_2$-O-); 1,91-1,04 (m, 6 H, -CH$_2$-CH$_2$-CH$_2$-NH, CH$_2$-CH$_2$-NH, O=C-(NH)CH-CH$_2$-).
IR: 3298*wb*, 2881*s*, 1657*m*, 1529*w*, 1466*w*, 1342*m*, 1279*w*, 1240*m*, 1103*s*, 962*m*, 843*m*.

Beispiel 19: Nachträgliche Modifizierung von Polylysin

7,0g festes Polylysin nach Beispiel 9 wurde in 50 ml Essigsäureanhydrid suspendiert und die Mischung 6 Stunden unter Rückfluß gekocht. Anschließend wurde das Lösemittel am Rotationsverdampfer bei im Vakuum entfernt. Die Ausbeute war quantitativ.
$^1$H-NMR (400MHz, CDCl$_3$): 4,39 (m, 1H, O=C-CH-NH-); 3,92 ((m, 1H, O=C-CH-NH-); 3,25 (m, 2H, -CH$_2$-NH); 2,87 (m, 2H, -CH$_2$-NH); 1,98 (s, 3H (O)C-CH$_3$); 1,96 (s, 3H (O)C-CH$_3$); 1,75 (mb, 1 H, -CH-CH$_2$-CH$_2$-); 1,48 (mb, 2H, CH$_2$-CH$_2$-NH); 1,31 (mb, 2 H, -CH$_2$-CH$_2$-CH$_2$-).
IR: 2931*s*, 2761*w*, 2646*w*, 2114*w*, 1635*m*, 1566*m*, 1490*m*, 1350*m*, 1304*m*, 1241*w*, 1149*m*, 1095*m*, 1026*s*, 980*m*, 895*m*, 825*w*, 717*m*.

Beispiel 20: Nachträgliche Modifizierung von Polylysin

1,5g festes Polylysin nach Beispiel 9 wurde in 10 ml Trifluoressigsäureanhydrid suspendiert und die Mischung 6 Stunden unter Rückfluß gekocht. Anschließend wurde das Lösemittel am Rotationsverdampfer bei 40 °C im Vakuum entfernt. Anschließend wurde das Polymer im Hochvakuum bei < 0,1 mbar bei Raumtemperatur getrocknet.

Beispiel 22: Modifiziertes Polylysin als Transport-Reagenz

Es wurde eine Lösung von 41,8 mg Kongorot in 1 Liter Wasser (6*10$^{-5}$ mol/l) hergestellt. 5 ml dieser Farbstoff-Lösung wurden mittels Pipette langsam auf 5 ml einer in einem Schnappdeckelglas befindlichen Lösung von 50 mg Lysin-Polymer aus Beispiel 16 in 5 ml Chloroform gegeben. Es bildeten sich zwei Phasen, wobei sich der Farbstoff in der oberen, wässrigen Phase befand. Das Schnappdeckelglas wurde geschlossen und kräftig geschüttelt. Nach erneuter Phasenseparation befand sich der Farbstoff in der unteren Chloroformphase.

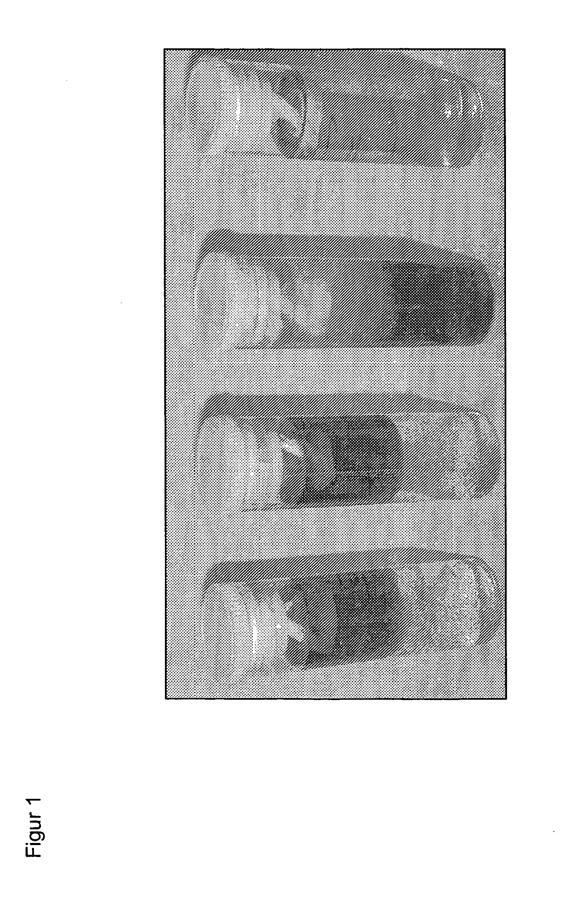

Die Ansätze sind in Figur 1 abgebildet:

Bei der oberen Phase handelt es sich um die Wasserphase, bei der unteren um die organische Chloroformphase. Ganz links: Wasser mit Farbstoff - oben, Chloroform - unten

2. Ansatz von links: Wasser mit Farbstoff - oben, Chloroform - unten, gemischt mit Stearinsäure (10 mg/ml), nach Schütteln und erneuter Phasenseparation

2. Ansatz von rechts: Wasser, Farbstoff, Chlorform und Stearinsäure-modifiziertes Polylysin (10 mg/ml), nach Schütteln und erneuter Phasenseparation

Ganz rechts: Chlorform und Stearinsäure-modifiziertes Polylysin (10 mg/ml)

Beispiel 23: Komplexierung von Polylysin mit Natriumdodecylsulfat (SDS)

1 g hochverzweigtes Polylysin nach Beispiel 9 (8,24 mmol NH$_2$ Equivalente) wurde in 30 ml MilliQ Wasser gelöst und der pH-Wert der Lösung mit 0,1 M HCl auf 3.5 eingestellt. Parallel dazu wurden 2,38 g Natriumdodecylsulfat (SDS, 8,26 mmol) in 100 ml Wasser gelöst und der pH-Wert der Lösung ebenfalls mit 0,1 M HCl auf 3.5 eingestellt. Die SDS Lösung wurde dann langsam unter Rühren in die wässrige Lösung des Polylysins gegeben, wobei die Reaktionsmischung sich trübte und sich ein Niederschlag bildete. Die Mischung wurde nach Beendigung der Zugabe noch weitere 15 Minuten gerührt und der Niederschlag dann abfiltriert. Der Filterrückstand wurde in 50 ml 1-Butanol gelöst und die butanolische Lösung dann langsam in 500 ml Wasser mit einem pH-Wert von 3,5 gegeben. Der gebildete Niederschlag wurde erneut abfiltriert und ausgiebig mit 2000 ml Wasser, eingestellt auf pH-Wert 3,5, gewaschen. Der weissgelbe Rückstand wurde im Exsikkator über $P_2O_5$ getrocknet. Die Ausbeute war quantitativ, der Beladungsgrad des Polylysins bezogen auf NH2-Gruppen wurde mit 95 % ermittelt.

$^1$H-NMR (400 MHz, $CD_3OD$, rt): 4.28 (br, 1 H, COCH(R)NH), 4.00 (t, 2 H, $J$= 6.92 Hz, SDS-C(1)$H_2$), 3.89 (br, 1 H, COC$\underline{H}$(R)NH), 3.23 (m, 2H, $CH_2$-NH), 2.98 (m, 2 H, $CH_2$-$NH_2$), 1.86 (br m, 2 H, COCH($CH_2$)NH), 1.66 (q, 2 H, $J$ = 6.78 Hz, SDS-C(2)$H_2$), 1.58 (br m, 2 H, $CH_2$- $CH_2$-NH), 1.38 (br m, 2 H, SDS-C(3)$H_2$), 1.35-1.20 (br m, 18 H, $CH_2$-$CH_2$-$CH_2$, SDS-C(4)-SDS-(C11)$H_2$), 0.90 (d, 3 H, $J$= 6.76 Hz, SDS-C(12)$H_2$). $^{13}$C-NMR (100.6 MHz, $CD_3OD$, rt): 168.4 ($\underline{C}$OCH(R) NH), 67.36 (SDS-C(1)), 52.59 (CO$\underline{C}$H(R)NH), 52.37 (CO$\underline{C}$H(R)NH), 38.45 ($\underline{C}H_2$-NH), 38.20 ($\underline{C}H_2$-NH), 31.14 (SDS-C (2)), 30.32 (COCH($\underline{C}H_2$)NH), 30.12 (COCH($\underline{C}H_2$)NH), 28.88, 28.83, 25.55, 28.52 (7 x SDS-C(3) - SDS-C(9)), 29.74 ($\underline{C}H_2$- $CH_2$-NH), 25.01 (SDS-C(10)), 21.80 (SDS-C(11)), 21.36 ($CH_2$-$\underline{C}H_2$- $CH_2$), 20.99 ($\underline{C}H_2$-$CH_2$- $CH_2$), 12.54 (SDS-C(12)).

Beispiel 24: Komplexierung von Polylysin mit Natriumoctylsulfat (SOS)

Es wurde verfahren wie unter Beispiel 23, nur dass anstelle von SDS 1,92 g Natriumoctylsulfat (SOS) verwendet wurden.

Die Ausbeute an weiß-gelbem Feststoff war quantitativ, der Beladungsgrad bezogen auf NH2-Gruppen wurde mit 90 % bestimmt.

$^1$H-NMR (400 MHz, $CD_3OD$, rt): 4.29 (br, 1 H, COC$\underline{H}$(R)NH), 4.00 (d, 2 H, $J$= 5.46 Hz, SOS-C(1)$H_2$), 3.89 (br, 1 H, COC$\underline{H}$(R)NH), 3.24 (m, 2 H, $CH_2$-NH), 2.98 (m, 2 H, $CH_2$-$NH_2$), 1.88 (br m, 2 H, COCH($CH_2$)NH), 1.67 (br m, 2 H, SOS-C(2)$H_2$), 1.60 (br m, 2 H, $CH_2$- $CH_2$-NH), 1.40 (br m, 2 H, $CH_2$-$CH_2$- $CH_2$), 1.32 (br m, 10 H, SOS-C(3)$H_2$-SOS-(C7)$H_2$), 0.91 (d, 3 H, $J$ = 4.75 Hz, SOS-C(8)$H_2$).

**Patentansprüche**

1. Verwendung von modifizierten Polylysinen, erhältlich durch Umsetzung eines beliebig erhaltenen Polylysins mit einem gewichtsmittleren Molekulargewicht $M_w$ von mehr als 7.500 Da und bis zu 750.000 Da mit mindestens einer Säuregruppen enthaltenden Verbindung, wobei diese Säuregruppen enthaltende Verbindung mindestens eine saure Gruppe, ausgewählt aus der Gruppe bestehend aus Carboxyl-, Sulfonsäure-, Sulfat- und Phosphonsäuregruppen, aufweist und mindestens einen Substituenten trägt, ausgewählt aus der Gruppe bestehend aus linearen oder verzweigten Alkylresten, gegebenenfalls substituierten Cycloalkylresten und gegebenenfalls substituierten Arylresten, die jeweils bis zu 20 Kohlenstoffatome aufweisen können, als Haftvermittler, Thixotropiermittel, Löslichkeitsvermittler (Solubilisatoren), als Phasentransferreagenz für in Wasser unlösliche Chemikalien, als Phasentransferreagenz für in Wasser lösliche Chemikalienoder als Surface-Modifier.

2. Verwendung nach Anspruch 1, **dadurch gekennzeichnet, daß** die Säuregruppen enthaltende Verbindung ausgewählt ist aus der Gruppe bestehend aus Acrylsäure, Maleinsäure und deren Derivaten.

3. Verwendung nach Anspruch 1, **dadurch gekennzeichnet, daß** saure die Säuregruppen enthaltende Verbindung ausgewählt ist aus der Gruppe bestehend aus Octansäure, Nonansäure, Decansäure, Dodecansäure, Hexadecansäure, Stearinsäure, Ölsäure, Linolsäure, Linolensäure, Octansulfonsäure, Dodecansulfonsäure, Stearylsulfonsäure, Oleylsulfonsäure, Camphersulfonsäure, Cyclododecylsulfonsäure, p-Toluolsulfonsäure, Benzolsulfonsäure, 4-Hexylbenzolsulfonat, 4-Octylbenzolsulfonat, 4-Decylbenzolsulfonat und 4-Dodecylbenzolsulfonat oder deren Li-, Na-, K-, Cs-, Ca- oder Ammoniumsalze.

4. Verwendung nach Anspruch 1, **dadurch gekennzeichnet, daß** die Alkyl-, Cycloalkyl-, oder Arylreste 6 bis 20 Kohlenstoffatome aufweisen.

5. Verwendung gemäß einem der vorstehenden Ansprüche, **dadurch gekennzeichnet, daß** man das Polylysin mit hydrophoben und hydrophilen Agenzien gleichzeitig modifiziert.

6. Verwendung gemäß einem der vorstehenden Ansprüche, **dadurch gekennzeichnet, daß** das in die Modifizierung eingesetzte Polylysin einen Gehalt an primären, sekundären oder tertiären, freien oder protonierten Aminogruppen, berechnet als $NH_2$, von 1 bis 21,9 Gew% aufweist.

7. Verwendung gemäß einem der vorstehenden Ansprüche, **dadurch gekennzeichnet, daß** das in die Modifizierung eingesetzte Polylysin einen Gehalt an freien oder deprotonierten Säuregruppen, berechnet als COOH, von 0 bis 30 Gew% aufweist.

**Claims**

1. The use of modified polylysines obtainable by reacting an arbitrarily obtained polylysine having a weight-average molecular weight $M_w$ of more than 7500 Da and up to 750 000 Da with at least one compound containing acid groups, said compound containing acid groups having at least one acidic group selected from the group consisting of carboxylic, sulfonic-acid, sulfate, and phosphonic-acid groups and carrying at least one substituent selected from the group consisting of linear or branched alkyl radicals, optionally substituted cycloalkyl radicals, and optionally substituted aryl radicals, which in each case may have up to 20 carbon atoms, as adhesion promoters, thixotropic agents, solubilizers, phase transfer reagents for water-insoluble chemicals, phase transfer reagents for water-soluble chemicals, or surface modifiers.

2. The use according to claim 1, wherein the compound containing acid groups is selected from the group consisting of acrylic acid, maleic acid, and derivatives thereof.

3. The use according to claim 1, wherein the compound containing acid groups is selected from the group consisting of octanoic acid, nonanoic acid, decanoic acid, dodecanoic acid, hexadecanoic acid, stearic acid, oleic acid, linoleic acid, linolenic acid, octanesulfonic acid, dodecanesulfonic acid, stearylsulfonic acid, oleylsulfonic acid, camphorsulfonic acid, cyclododecylsulfonic acid, p-toluenesulfonic acid, benzenesulfonic acid, 4-hexylbenzenesulfonate, 4-octylbenzenesulfonate, 4-decylbenzenesulfonate, and 4-dodecylbenzenesulfonate, or their Li, Na, K, Cs, Ca, or ammonium salts.

4. The use according to claim 1, wherein the alkyl, cycloalkyl, or aryl radicals have 6 to 20 carbon atoms.

5. The use according to any of the preceding claims, wherein the polylysine is simultaneously modified with hydrophobic and hydrophilic agents.

6. The use according to any of the preceding claims, wherein the polylysine used in the modification contains from 1 to 21.9 wt% of primary, secondary, or tertiary, free or protonated amino groups, calculated as $NH_2$.

7. The use according to any of the preceding claims, wherein the polylysine used in the modification contains from 0 to 30 wt% of free or deprotonated acid groups, calculated as COOH.

**Revendications**

1. Utilisation de polylysines modifiées, pouvant être obtenues par mise en réaction d'une polylysine obtenue de manière quelconque d'un poids moléculaire moyen en poids $M_w$ supérieur à 7 500 Da et de jusqu'à 750 000 Da avec au moins un composé contenant des groupes acides, ce composé contenant des groupes acides comprenant au moins un groupe acide choisi dans le groupe constitué par les groupes carboxyle, acide sulfonique, sulfate et acide phosphonique et portant au moins un substituant choisi dans le groupe constitué par les radicaux alkyle linéaires ou ramifiés, les radicaux cycloalkyle éventuellement substitués et les radicaux aryle éventuellement substitués, qui peuvent chacun comprendre jusqu'à 20 atomes de carbone, en tant que promoteur d'adhésion, agent thixotropique, promoteur de solubilité (solubilisant), en tant que réactif de transfert de phase pour produits chimiques insolubles dans l'eau, en tant que réactif de transfert de phase pour produits chimiques solubles dans l'eau ou en tant que modificateur de surface.

2. Utilisation selon la revendication 1, **caractérisée en ce que** le composé contenant des groupes acides est choisi dans le groupe constitué par l'acide acrylique, l'acide maléique et leurs dérivés.

3. Utilisation selon la revendication 1, **caractérisée en ce que** le composé contenant des groupes acides est choisi dans le groupe constitué par l'acide octanoïque, l'acide nonanoïque, l'acide décanoïque, l'acide dodécanoïque, l'acide hexadécanoïque, l'acide stéarique, l'acide oléique, l'acide linoléique, l'acide linolénique, l'acide octanesulfonique, l'acide dodécanesulfonique, l'acide stéarylsulfonique, l'acide oléylsulfonique, l'acide camphresulfonique, l'acide cyclododécylsulfonique, l'acide p-toluènesulfonique, l'acide benzènesulfonique, le sulfonate de 4-hexylbenzène, le sulfonate de 4-octylbenzène, le sulfonate de 4-décylbenzène et le sulfonate de 4-dodécylbenzène ou leurs sels de Li, Na, K, Cs, Ca ou d'ammonium.

4. Utilisation selon la revendication 1, **caractérisée en ce que** les radicaux alkyle, cycloalkyle ou aryle comprennent 6 à 20 atomes de carbone.

5. Utilisation selon l'une quelconque des revendications précédentes, **caractérisée en ce que** la polylysine est modifiée simultanément avec des agents hydrophobes et hydrophiles.

6. Utilisation selon l'une quelconque des revendications précédentes, **caractérisée en ce que** la polylysine utilisée dans la modification présente une teneur en groupes amino primaires, secondaires ou tertiaires, libres ou protonés, calculée en tant que $NH_2$, de 1 à 21, 9% en poids.

7. Utilisation selon l'une quelconque des revendications précédentes, **caractérisée en ce que** la polylysine utilisée dans la modification présente une teneur en groupes acides libres ou déprotonés, calculée en tant que COOH, de 0 à 30 % en poids.

Figur 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2003064452 A, von Klok
- WO 0071600 A
- JP 11255892 A

**In der Beschreibung aufgeführte Nicht-Patentliteratur**

- **P.J. FLORY.** *J. Am. Chem. Soc.,* 1952, vol. 74, 2718
- **H. FREY et al.** *Chemistry - A European Journal,* 2000, vol. 6 (14), 2499
- **H. FREY et al.** *Acta Polym.,* 1997, vol. 48, 30
- *Macromolecules,* 2002, vol. 35, 8718-8723
- **RODRIGUEZ-HERNÄNDEZ et al.** *Biomacromolecules,* 2003, vol. 4, 249-258
- **BIRCHALL et al.** *Chem. Commun,* 1998, 1335-1336
- **T.L. MENZ ; T. CHAPMAN.** *Polym. Prep.,* 2003, vol. 44 (2), 842-743
- **PLAQUET ; MITARBEITER.** *Biochimie,* 1975, vol. 57, 1395-1396
- **HARADA.** *Bull. Chem. Soc. Japan,* 1959, vol. 32, 1007-1008
- **ROHLFING ; MITARBEITER.** *Archives of Biochemistry and Biophysics,* 1969, vol. 130, 441-448
- **FOX et al.** *BioSystems,* 1976, vol. 8, 40-44